# EUROPEAN PATENT APPLICATION

(11) **EP 4 609 853 A1**
(43) Date of publication of application: **03.09.2025**
(21) Application number: 23882046.8
(22) Date of filing: 27.09.2023
(51) Int. Cl.: A61K 9/00, A61K 31/05, A61K 31/185, A61K 33/00, A61K 33/38, A61K 47/36, A61P 17/02

(54) **ABSORBABLE COMPOSITION FOR REGENERATION OF SKIN LESIONS**

(30) Priority: 26.10.2022 MX 2022013483
(71) Applicant: Universidad de Guadalajara, 44100 Guadalajara, Jalisco (MX); Pérez Rosas, Fernanda, Guadalajara, Jalisco, 44890 (MX)
(72) Inventor: LÓPEZ DE LA MORA, David Alejandro, Zapopan, Jalisco, 45120 (MX); HERNÁNDEZ JIMÉNEZ, María Fernanda, Tonalá, Jalisco, 45400 (MX); FERNANDEZ AVILA, Leonardo, Guadalajara, Jalisco, 44630 (MX); GARCÍA BECERRA, Natalia, Guadalajara, Jalisco, 44648 (MX); PÉREZ ROSAS, Fernanda, Guadalajara, Jalisco, 44890 (MX)
(74) Representative: De Clercq & Partners
(86) International application number: PCT/IB2023/059634
(87) International publication number: WO 2024/089495

(57) **Abstract**

An absorbable composition for the regeneration of skin lesions, comprising: chitosan; an organic solvent; metal nanoparticles; resveratrol; taurine; and at least one pharmaceutically acceptable excipient.

## Description

### FIELD OF THE INVENTION

The present invention relates to medical materials, more particularly, to an absorbable composition for the regeneration of skin lesions.

### BACKGROUND OF THE INVENTION

Wounds that have not progressed through the normal healing process and are open for more than one month are classified as chronic wounds. There are various etiologies of chronic wounds, all of which pose a burden to the healthcare system. Patients suffering from diabetes and obesity are at high risk of developing chronic wounds. As such, the funding for research that directly addresses the study of chronic wounds is disproportionately low compared to the overall impact of chronic wounds as a healthcare problem. Chronic wounds are primarily observed in the elderly population. In the United States, 3% of the population over the age of 65 has open wounds. By 2020, the United States government estimated that the elderly population exceeded 55 million, suggesting that chronic wounds will continue to be an increasingly persistent problem in the elderly population or those with comorbidities.

The wound dressing market is one of the largest in the world, as there is a significant demand for wound care products. Globally, the annual cost of wound care averaged $2.8 billion in 2014. A 2018 market research report predicts that the global wound care products market will exceed $15 billion by 2022, and the advanced wound care market targeting surgical wounds and chronic ulcers is expected to exceed $22 billion by 2024, driven by technological advancement, rising incidence of chronic wounds, increasing government support, and a growing geriatric population (Sen C. K. (2019). Human Wounds and Its Burden: An Updated Compendium of Estimates. Advances in wound care, 8(2), 39-48. https://doi.org/10.1089/wound.2019.0946).

Traditional gauze bandages for wound treatment are prepared primarily from raw materials such as cotton and hemp, and their manufacturing process is simple and low-cost. However, traditional gauze dressings cannot maintain the moist environment required for wound healing. Their easy adhesion to wound tissue causes secondary mechanical problems, and their use can even cause injury during dressing changes. Another problem stemming from their use is their lack of antiseptic properties, which leads to contamination of the wound surface, unpleasant odor, and causes wound complications by impeding wound healing. With the advancement of modern science and technology, new medical dressings are continually emerging, and their clinical efficacy is also continually improving.

Currently, research is focusing on the development of new products for chronic wound regeneration. In this regard, document MX319756 describes the formulation of a wound healing dressing comprising a blend of a high-molecular-weight silicone elastomer crosslinked polymer and a silicone oil, wherein the silicone elastomer crosslinked polymer is in a volatile fluid. Although the main objective of the invention is to provide greater protection to the wound by being a substantial dressing, it is not mentioned that it is absorbable or degradable, which has as a side effect the possible adhesions derived from its application and its subsequent removal at the end of the treatment for its proper disposal as hazardous sanitary waste.

Therefore, there is a need to develop new absorbable compositions that do not generate hazardous medical waste after use, are easy to apply, and avoid the discomfort associated with their removal. Furthermore, it is important that absorbable compositions utilize active ingredients that prevent wound contamination and even reduce wound healing time by controlling inflammation.

### OBJECTIVES OF THE INVENTION

Taking into account the shortcomings of the prior art, one object of the present invention is to provide an absorbable composition for the regeneration of skin lesions.

It is also another object of the present invention to provide an absorbable composition that reduces wound recovery time by reducing the likelihood of infection.

Another object of the present invention relates to an absorbable composition for the regeneration of skin lesions that is easy to apply and avoids the discomfort associated with its removal.

Another object of the present invention relates to an absorbable composition for the regeneration of skin lesions that does not generate hazardous medical waste after use.

### SUMMARY OF THE INVENTION

To this end, a first aspect of the present invention relates to an absorbable composition for the regeneration of skin lesions, comprising: chitosan; an organic solvent; metal nanoparticles; resveratrol; taurine; and at least one pharmaceutically acceptable excipient.

A second aspect of the present invention relates to an absorbable composition for use in the regeneration of skin lesions, wherein the composition comprises: chitosan, an organic solvent, metal nanoparticles, resveratrol, taurine, and at least one pharmaceutically acceptable excipient; and wherein the composition is adapted to be administered topically.

A third aspect of the present invention relates to the use of an absorbable composition comprising: chitosan, an organic solvent, metal nanoparticles, resveratrol, taurine, and at least one pharmaceutically acceptable excipient for the manufacture of a topical medicament for the regeneration of skin lesions.

### BRIEF DESCRIPTION OF THE FIGURES

Novel aspects which are considered characteristic of the present invention will be established with particularity in the appended claims. However, some embodiments, features and some objects and advantages thereof will be better understood in the detailed description, when read in connection with the attached drawings, in which:
Figure 1 illustrates the growth kinetics of *Escherichia coli,* where P is the positive control (silver nanoparticles); N is the negative control (without any treatment); and T is the treatment (absorbable composition).
Figure 2 illustrates the growth kinetics of *Escherichia coli,* where P is the positive control (silver nanoparticles); N is the negative control (without any treatment); and T is the treatment (absorbable composition).
Figure 3 illustrates the growth kinetics of *Escherichia coli,* where P is the positive control (silver nanoparticles); N is the negative control (without any treatment); and T is the treatment (absorbable composition).
Figure 4 illustrates the regeneration progression of a second-degree burn after the administration of the absorbable composition, where A shows the untreated burn wound; B shows the wound treated with the absorbable composition on the fifth day of treatment; and C shows the wound treated with the absorbable composition on the seventh day of treatment.
Figure 5 illustrates the regeneration progression of a diabetic foot wound after administration of the absorbable composition, where A shows the untreated wound; B shows the wound treated with the absorbable composition on the fifth day of treatment; and C shows the wound treated with the absorbable composition on the tenth day of treatment.

### DETAILED DESCRIPTION OF THE INVENTION

The present invention exhibits certain advantages over the prior art, among which it can be mentioned that the absorbable composition decreases the recovery time of a wound by reducing the likelihood of infection after its use, and promotes healing by reducing inflammation. Furthermore, it's easy to apply and absorb, so it doesn't need to be removed, avoiding the discomfort of conventional dressings. It also doesn't generate hazardous medical waste.

To this end, a first aspect of the present invention relates to an absorbable composition for the regeneration of skin lesions, comprising: chitosan; an organic solvent; metal nanoparticles; resveratrol; taurine; and at least one pharmaceutically acceptable excipient.

Preferably, the absorbable composition for the regeneration of skin lesions comprises: chitosan in a concentration of between 0.5 and 10% (w/w); an organic solvent in a concentration of between 0.01% to 1% (w/w); metal nanoparticles in a concentration of between 0.001% and 10%; resveratrol in a concentration of between 0.01% and 10%; taurine in a concentration of between 0.01% and 10%; and at least one pharmaceutically acceptable excipient in a concentration of between 50% and 99.5% (w/w).

Preferably, the organic solvent is an organic acid. More preferably, the organic acid is acetic acid.

Preferably, the metal nanoparticles are silver nanoparticles with a particle size of between 10 and 15 nm.

Preferably, the chitosan has a purity level of 99%.

Preferably, the absorbable composition for the regeneration of skin lesions further comprises at least one gelling agent in a concentration of between 0.5% and 10% (w/w) to obtain a semisolid topical composition in the form of a gel, cream, or ointment.

More preferably, the gelling agent is selected from the group consisting of agar-agar and polyvinyl alcohol.

A second aspect of the present invention relates to an absorbable composition for use in the regeneration of skin lesions, wherein the composition comprises: chitosan, an organic solvent, metal nanoparticles, resveratrol, taurine, and at least one pharmaceutically acceptable excipient; and wherein the composition is adapted to be administered topically.

A third aspect of the present invention relates to the use of an absorbable composition comprising: chitosan, an organic solvent, metal nanoparticles, resveratrol, taurine, and at least one pharmaceutically acceptable excipient for the manufacture of a topical medicament for the regeneration of skin lesions.

The present invention will be better understood from the following Examples, which are presented solely for illustrative purposes to allow a full comprehension of the preferred embodiments of the present invention, without implying that there are no other non-illustrated embodiments that may be put into practice based on the detailed description above.

### EXAMPLE 1

In this study, the synergistic effect of resveratrol in combination with taurine was evaluated in the absorbable composition for the regeneration of skin diseases according to the present invention. The study included a cell viability analysis using the VitroSkin^{®} Reconstituted Human Epidermis (RHE) model, which was based on the metabolic reduction of 3-(4,5-dimethylthiazol-2-yl)-2,5-diphenyltetrazolium bromide (MTT) by the mitochondrial enzyme succinate dehydrogenase to a blue-colored compound (formazan), allowing the determination of mitochondrial functionality of the treated cells. The VitroSkin^{®} model from the Alternative Research Laboratory (LIALT) uses normal human newborn epidermal keratinocytes (HEKn; one of the most abundant cells in the skin, surface area 0.63 cm²), which were cultured in special inserts with nutrients for a period of 12 days.

Four different treatments (M) were prepared in gel form for the study, as shown in Table 1.

**TABLE 1**

| **ID** | **Sample Description** | **Composition of the sample % (w/w)** |
|---|---|---|
| M-35 | Taurine + Resveratrol Sample | 2% chitosan; 0.019% acetic acid; 2% taurine; 1% resveratrol; and 0.03% silver nanoparticles. |
| M-36 | Taurine with No Resveratrol Sample | 2% chitosan; 5% acetic acid; 2% taurine; and 0.03% silver nanoparticles. |
| M-37 | Taurine Sample with No Resveratrol | 2% chitosan; 5% acetic acid; 1% resveratrol; and 0.03% silver nanoparticles. |
| M-38 | No Taurine No Resveratrol Sample | 2% chitosan; 5% acetic acid; and 0.03% silver nanoparticles. |

The tissues were exposed to the prepared gel samples (0.63 cm²) directly on the surface of the reconstituted human epidermis model (n = 3) and incubated at 37°C for 60 minutes. On the other hand, Untreated Control tissues (UCT) were exposed in the same manner with the purpose of evaluating a response related to the applied dose. The tissues were then thoroughly rinsed, dried to remove test substances, and transferred to fresh media for 24 hours. After this time, the media was changed again and incubated again for 18 hours prior to the cell viability assay. The cell viability assay in the RhE models was measured by the enzymatic conversion of the vital dye MTT (3-(4,5-dimethylthiazol-2-yl)-2,5-diphenyltetrazolium bromide, thiazolyl blue; CAS number 298-93-1], in a blue formazan salt that was quantitatively measured after tissue extraction. The absorbance of the resulting solution was measured at 570 nm using a Thermo Fisher Scientific Varioskan Flash^{®} microplate reader. Absorbance values were captured using Skanlt^{®} Software 2.4.5, and the statistical analysis was performed using the GraphPadPrism v6^{®} program.

The absolute Optical Density of untreated control tissues (UCT) (treated with sterile DPBS) in the MTT assay is an indicator of tissue viability obtained in the testing laboratory after transportation procedures, storage, and under specific conditions of use. The assay met the acceptance criterion if the average OD570 of the CST tissues was >0.8 and <2.5. Since the skin irritation potential in each assay is predicted from the average viability determined in two individual tissues, the tissue replicate variability should be acceptably low. The assay met the acceptance criterion if the SD calculated from the individual tissue viabilities of the two identically treated replicates was ≤18%, which is a range recommended by the EU test method guideline B.46. The cell viability results for the treatments evaluated in the LIALT VitroSkin EHR model are shown in Table 2. The average OD value of n=2 EHRs per treatment evaluated was calculated and presented as a % of the untreated control ± % SD.

**TABLE 2**

| **ID** | **Sample Description** | **% Cellular Viability¹** | **DS %²** |
|---|---|---|---|
| UCT | Untreated Control Tissues | 100.00 | 0.27 |
| M-35 | Taurine + Resveratrol Sample | 112.79 | 3.27 |
| M-36 | Taurine with No Resveratrol Sample | 114.61 | 0.24 |
| M-37 | Taurine Sample with No Resveratrol | 107.53 | 3.38 |
| M-38 | No Taurine No Resveratrol Sample | 99.77 | 1.53 |

| | | | |
|---|---|---|---|
| ¹average of 2 replicates ²SD: Standard deviation | | | |

The results show that M-35 has a high percentage of cell viability, so the use of resveratrol in the composition does not significantly affect it. Although the results show that cell viability is better in M-36, the inclusion of resveratrol in the composition is important, as its use decreases inflammatory activity by inhibiting the synthesis of proinflammatory markers.

### EXAMPLE 2

In this assay, the antimicrobial effect of the absorbable composition for the regeneration of skin diseases according to the present invention was evaluated.

The absorbable composition was evaluated against three different microorganisms typically present in an infected wound. The microorganisms were: *Escherichia coli, Staphylococcus aureus* and *Pseudomonas aeruginosa.* In addition, silver nanoparticles were used as a positive control.

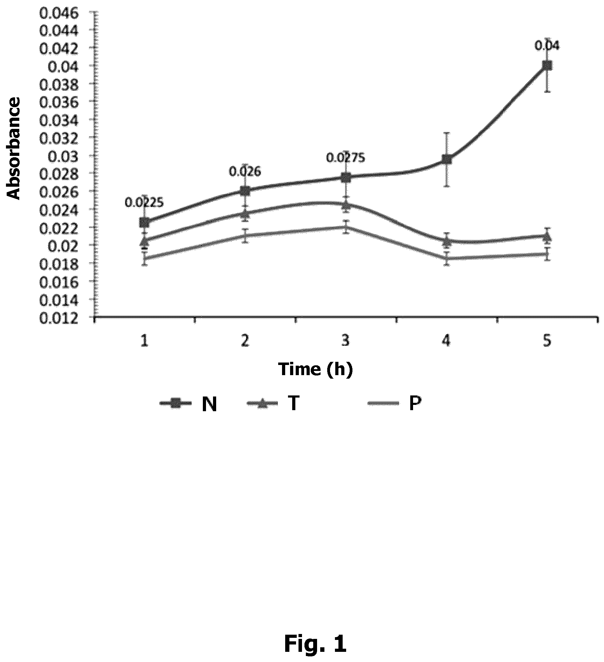

The microorganisms were cultured in culture broth enriched for bacterial growth against the absorbable composition (treatment), silver nanoparticles as a positive control, and only the culture broth as a negative control. The microorganisms were incubated under constant shaking conditions and at a temperature of 42°C for 5 hours. To monitor growth kinetics, the samples were sampled every hour and the absorbance was determined by spectrophotometry to quantify growth. Figure 1 illustrates the growth kinetics of *Escherichia coli,* where P is the positive control (silver nanoparticles); N is the negative control (no treatment); and T is the treatment (absorbable composition).

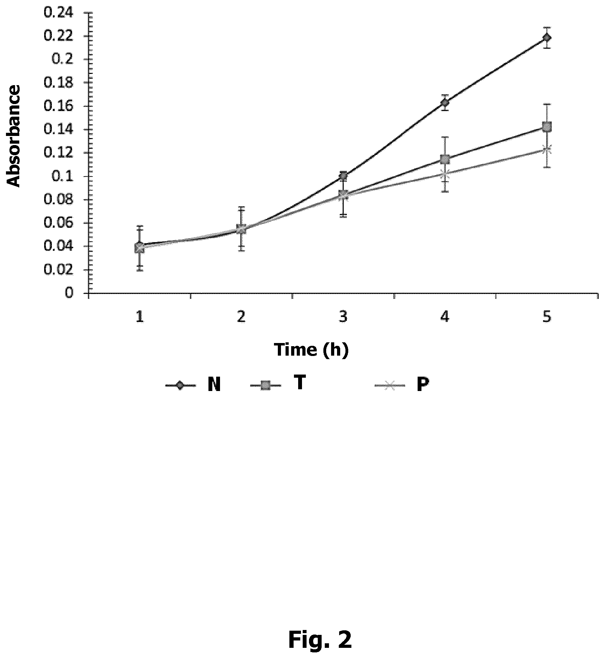

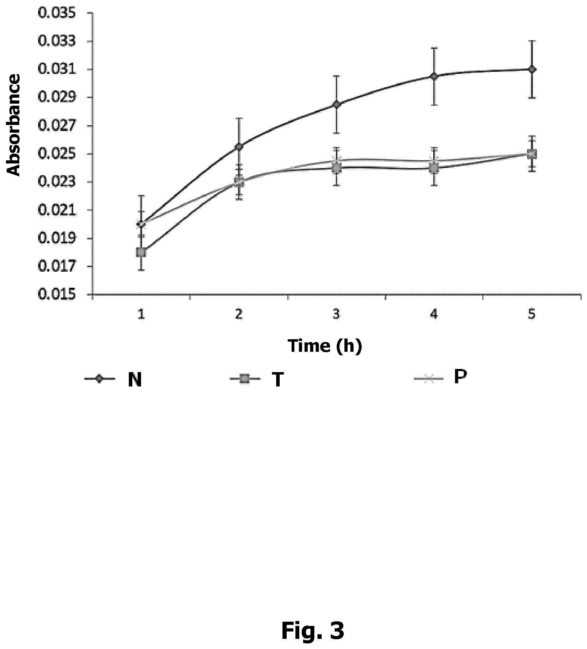

The results of this assay are illustrated in Figure 2, which shows the growth kinetics of *Staphylococcus aureus,* where P is the positive control (silver nanoparticles); N is the negative control (no treatment); and T is the treatment (absorbable composition). Likewise, Figure 3 illustrates the growth kinetics of *Pseudomonas aeruginosa,* where P is the positive control (silver nanoparticles); N is the negative control (without any treatment); and T is the treatment (absorbable composition).

In all kinetics, the inhibition of the absorbable membrane was similar to the positive control where only silver nanoparticles were used. This suggests that the use of silver nanoparticles in the absorbable composition for the regeneration of skin diseases is not incompatible, as the antimicrobial effect is maintained.

### EXAMPLE 3

To demonstrate the effectiveness of the absorbable composition for the regeneration of skin diseases, trials were conducted on patients with chronic wounds. The composition comprised: chitosan at a concentration of at least 2% (w/w); acetic acid at a concentration of at least 1% (w/w); resveratrol at a concentration of at least 1% (w/w); taurine at a concentration of at least 1.5% (w/w); silver nanoparticles at a concentration of at least 0.034% (w/w); and at least one pharmaceutically acceptable excipient at a concentration of at least 94.46% (w/w).

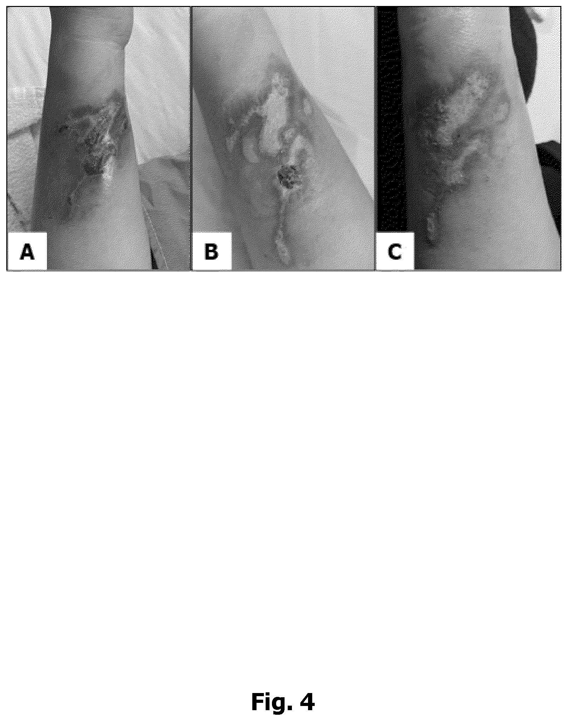

The first diabetic male patient showed up with a second-degree burn with no apparent progression for 3 weeks. Figure 4 shows the evolution of wound regeneration after the administration of the absorbable composition, wherein A shows the untreated burn wound; B shows the wound treated with the absorbable composition on the fifth day of treatment; and C shows the wound treated with the absorbable composition on the seventh day of treatment. It can be seen that the second-degree burn wound improved considerably during administration of the absorbable composition.

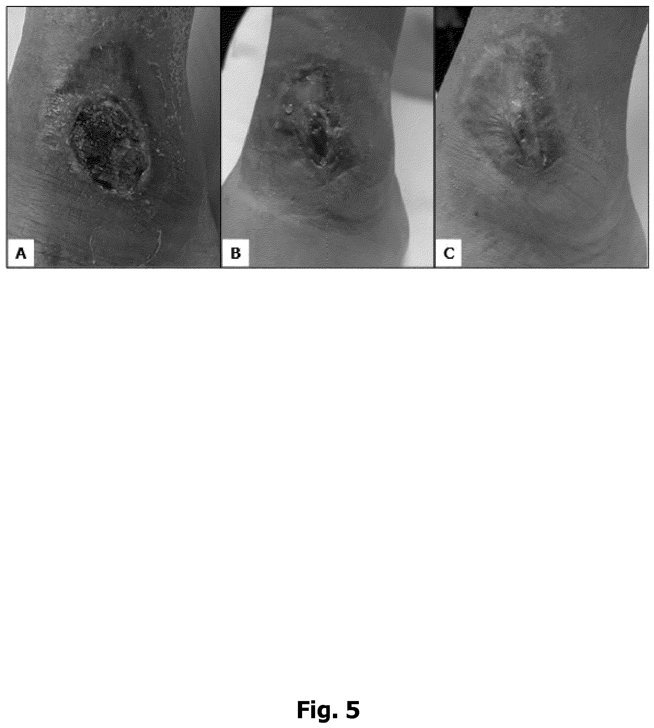

The second diabetic female patient had a chronic diabetic foot wound that had lasted 4 months. Figure 5 shows the evolution of wound regeneration after the administration of the absorbable composition, wherein A shows the untreated wound; B shows the wound treated with the absorbable composition on the fifth day of treatment; and C shows the wound treated with the absorbable composition on the tenth day of treatment. Similar to the first treated patient, it can be seen that the diabetic foot wound improved considerably during administration of the absorbable composition in the second patient.

In accordance with the foregoing, it can be seen that the absorbable composition for the regeneration of skin lesions has been designed for its application in the field of medicine, and it will be evident to any person skilled in the art that the embodiments of the invention, as described above and illustrated in the accompanying drawings, are merely illustrative and not limiting of the present invention, since numerous significant changes in its details are possible without departing from the scope of the invention. For example, it is possible to use the absorbable composition for the regeneration of skin lesions different from those shown in the previously described examples.

Therefore, the present invention should not be construed as restricted except as required by the prior art and by the scope of the appended claims.

## Claims

1. An absorbable composition, **characterized in that** it comprises: chitosan; an organic solvent; metal nanoparticles; resveratrol; taurine; and at least one pharmaceutically acceptable excipient.

2. The composition according to claim 1, further characterized because it comprises: chitosan at a concentration of between 0.5 and 10% (w/w); an organic solvent at a concentration of between 0.01% to 1% (w/w); metal nanoparticles at a concentration of between 0.001% and 10%; resveratrol at a concentration of between 0.01% and 10%; taurine at a concentration of between 0.01% and 10%; and at least one pharmaceutically acceptable excipient at a concentration of between 50% and 99.5% (w/w).

3. The composition according to claim 1, further characterized because the organic solvent is an organic acid.

4. The composition according to claim 1, further characterized because the organic acid is acetic acid.

5. The composition according to claim 1, further characterized because the metal nanoparticles are silver nanoparticles with a particle size of between 10 and 15 nm.

6. The composition according to claim 1, further characterized because the chitosan has a purity level of 99%.

7. The composition according to claim 1, further characterized because it additionally comprises at least one gelling agent at a concentration of between 0.5% and 10% (w/w).

8. The composition according to claim 1, further characterized because the gelling agent is selected from the group consisting of agar-agar and polyvinyl alcohol.

9. An absorbable composition for use in the regeneration of skin lesions, wherein the composition comprises: chitosan, an organic solvent, metal nanoparticles, resveratrol, taurine, and at least one pharmaceutically acceptable excipient; and wherein the composition is adapted to be administered topically.

10. The use of an absorbable composition comprising: chitosan, an organic solvent, metal nanoparticles, resveratrol, taurine, and at least one pharmaceutically acceptable excipient for the manufacture of a topical medicament for the regeneration of skin lesions.
